(19) Europäisches Patentamt
European Patent Office
Office européen des brevets

(11) Publication number : **0 454 863 B1**

(12) # EUROPEAN PATENT SPECIFICATION

(45) Date of publication of patent specification : **03.11.93 Bulletin 93/44**

(51) Int. Cl.$^5$ : **C07C 45/72,** C07C 49/17, C07C 49/172, C07C 49/245, C07C 323/22, C07D 335/02, C07D 309/06, C07D 307/46

(21) Application number : **90916804.9**

(22) Date of filing : **15.11.90**

(86) International application number : **PCT/JP90/01486**

(87) International publication number : **WO 91/07369 30.05.91 Gazette 91/12**

(54) **METHOD FOR THE PREPARATION OF BETA-HYDROXYKETONES.**

(30) Priority : **17.11.89 JP 297386/89**

(43) Date of publication of application : **06.11.91 Bulletin 91/45**

(45) Publication of the grant of the patent : **03.11.93 Bulletin 93/44**

(84) Designated Contracting States : **AT BE CH DE DK ES FR GB GR IT LI LU NL SE**

(56) References cited :
**EP-A- 0 323 915**
**WO-A-86/02065**
**CHEMICAL ABSTRACTS, vol. 94, 1981, Columbus, Ohio, US, see p. 638, column 2, abstract no. 120871x; & JP-A-80141429**

(73) Proprietor : **NIPPON SODA CO., LTD.**
**2-1, Ohtemachi 2-chome**
**Chiyoda-ku, Tokyo 100 (JP)**

(72) Inventor : **TSUKASHIMA, K. Takaoka Plant, Nippon Soda Co., Ltd**
**300, Mukainohonmachi**
**Takaoka-shi Toyama 933 (JP)**
Inventor : **KANEKO, Akira Takaoka Plant, Nippon Soda Co., Ltd.**
**300, Mukainohonmachi**
**Takaoka-shi Toyama 933 (JP)**

(74) Representative : **de Bruijn, Leendert C. et al**
**Nederlandsch Octrooibureau**
**Scheveningseweg 82 P.O. Box 29720**
**NL-2502 LS Den Haag (NL)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

Technical Field:

This invention relates to a method for the preparation of β-hydroxyketones represented by general formula (I)

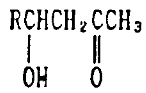

(I)

(where R is an alkyl group which may have a methyl group at the α position, an alkyl group substituted by alkylthio group which may have a methyl group at the α position, an alicyclic group which may have substituents at positions other than the α, β and β' positions, a heterocyclic group which may have substituents at positions other than the α, β and β' positions, and a phenyl group which may have an electron withdrawing group at the para position) (hereinafter referred to as Compound I). Compound I is extremely important as an intermediate for pharmaceuticals and agricultural chemicals.

Background Art:

Documents such as Ber. 40 4764 (1907) Ann. Chim.. (Paris) 6 406-86 (1951) describe that Compound I is conventionally synthesized by aldol type condensation of aldehyde and acetone using such a compound as NaOH as a catalyst. The yield is generally low, particularly when aldehyde with α hydrogen is used. A very excessive amount of acetone is used in many cases so that diacetone alcohol, which is a reaction product of acetone itself, is inevitably byproduced in a large amount.

In the method described in Japanese open patent No. Sho 55-141429 that aldehyde and alkaline metal salt of acetoacetic acid are reacted, triethylamine or trimethylamine, which is a water-soluble amine, is used as a catalyst. Therefore the method has industrially big problems of odor and waste water treatment. In the method described in Japanese open patent No. Sho 55-141429 the yield is good if aldehyde with two α hydrogen atoms is used. If aldehyde with no or one α hydrogen atom is used, the reaction is slow and yield is low. The purity is low so that the intended product must be purified by distillation if used as an industrial material.

An object of this invention is to provide methods for the preparation of β-hydroxyketones which do not use water-soluble amines having the problems of odor and waste water treatment and which are also applicable if aldehyde with no or one α hydrogen atom is used.

Disclosure of Invention:

The inventors earnestly studied reactions of aldehyde with aqueous solution of alkaline metal salt of acetoacetic acid in order to accomplish the said purpose, and found as a result that the reaction smoothly proceeds only in the pH range of 9 to 10, a mole ratio of aldehyde to alkaline metal salt of acetoacetic acid is important because the reactivity greatly differs depending on the structure of aldehyde, an addition of an appropriate amount of water-soluble organic solvent such as methanol, ethanol, glycerol or 1,4-dioxane makes the reaction faster, and a pH selection is needed when alkaline metal bicarbonate formed by the reaction is removed. Thus this invention has been completed.

Thus, this invention concerns a method for the preparation of β-hydroxyketones represented by general formula

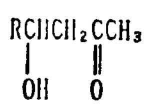

(where R is an alkyl group which may have a methyl group at the α position, an alkyl group substituted by alkylthio group which may have a methyl group at the α position, an alicyclic group which may have substituents at positions other than the α, β and β' positions, a heterocyclic group which may have substituents at positions other than the α, β and β' positions, and a phenyl group which may have an electron withdrawing group at the para position), by reacting aldehydes represented by general formula

RCHO (A)

(where R is as defined above) with aqueous solution of alkaline metal salt of acetoacetic acid represented by general formula

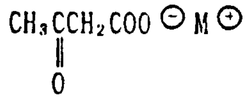

(B)

(where $M^{\oplus}$ is an alkali metal ion), which method comprises reacting (A) and (B) in a mole ratio of (A) to (B) of 1.0 to 1.5 if (A) has two a hydrogen atoms, 1.5 to 2.0 if (A) has one a hydrogen atom, 2.0 to 2.5 if (A) has no a hydrogen atom and 1.1 to 1.5 if R is a phenyl group with electron withdrawing group at the para position at a pH of 9 to 10, without using an amine catalyst.

Best Mode for Carrying Out the Invention:

The reaction in this invention smoothly proceeds only in a specific pH range: Good pH during the reaction is 9 to 10. If pH is lower than 9, the reaction becomes slow and the decomposition of alkaline metal salt of acetoacetic acid becomes relatively faster and hence the yield is low. If pH exceeds 10, the yield of the intended product is low because α, β-unsaturated ketones represented by general formula

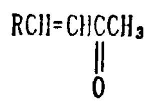

(where R is as defined above), which are dehydrated products of the intended product of β-hydroxyketones, are byproduced.

In the method of this invention, pH is adjusted with mineral acid or hydroxide alkaline aqueous solution, with no problems of waste water treatment.

The reaction of this invention is a two-step reaction represented by

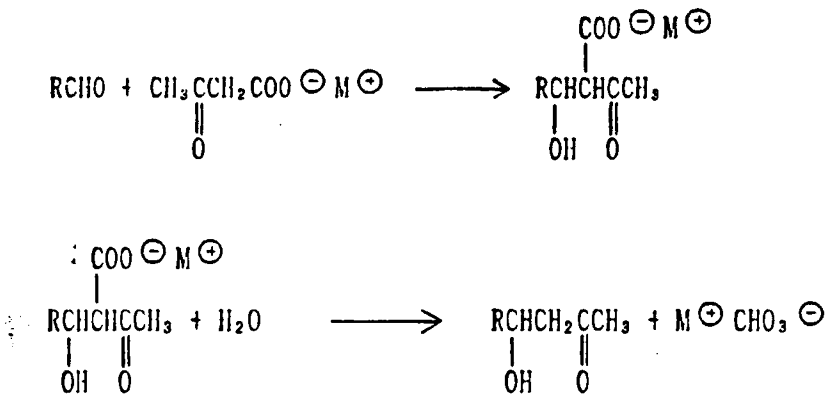

The reaction of the first step is an equilibrium reaction. When aldehyde with 2 α hydrogen atoms is used, the reaction of the first step smoothly proceeds and the reaction of the second step is rate-determining. However, in the case of aldehyde branched at the α position, the reaction of the first step is slow because of its steric hindrance, and the equilibrium is shifted to the material side when compared with tliat of aldehyde not branched at the α position. Under the conditions that the reaction of the second step smoothly proceeds, decomposition of alkaline metal salt of acetoacetic acid into acetone and alkaline metal bicarbonate is accelerated. Therefore the yield is low. A mole ratio of aldehyde to alkaline metal salt of acetoacetic acid is an important factor in order to solve those problems. For instance, a mole ratio of alkaline metal salt of acetoacetic acid can be small when aldehyde with two α hydrogen atoms such as 3-ethylthiobutanal is used. In the case of aldehyde with great steric hindrance such as 3-tetrahydrothiopyran carbaldehyde, the reaction does not proceed smoothly if a mole ratio of alkaline metal salt of acetoacetic acid is small, and the yield is low. It is therefore necessary to use an

appropriate amount of alkaline metal salt of acetoacetic acid according to the steric structure of aldehyde.

In other words, the mole ratio of aldehydes represented by general formula (A) to alkaline metal salt of acetoacetic acid represented by general formula (B) is 1.0 to 1.5 if aldehydes represented by general formula (A) have two α hydrogen atoms. 1.5 to 2.0 if they have one α hydrogen atom, 2.0 to 2.5 if they have no α hydrogen atom and 1.1 to 1.5 if R is a phenyl group with electron withdrawing group at the para position. It goes without saying that use of excessive amount of alkali metal salt of acetoacetic acid is not preferable in economy, because the excessive portion is decomposed into acetone and alkali metal bicarbonate, though there is no effect on the yield of purity.

In the method of this invention, an addition of appropriate amount of water-soluble organic solvent such as methanol, ethanol, glycerol or 1.4-dioxane makes the reaction faster. An addition of excessive amount greatly reduces the polarity of the reaction system, resulting in accelerated decomposition of alkaline metal salt of acetoacetic acid and low yield. A preferable addition of water-soluble organic solvent such as methanol is less than 300ml to one mole of aldehyde

In this invention, alkali metal bicarbonate is formed by the reaction and deposited as crystal. In Japanese open patent No. Sho 55-141429, the bicarbonate crystal deposited after the reaction is decomposed by using a mineral acid such as concentrated hydrochloric acid to make the pH 4. When β-hydroxyketone branched at the γ position is used, if the reaction solution is made acidic, the remaining material aldehyde reacts with formed β-hydroxyketone to form byproducts. Hence the yield becomes extremely low. Because of this, after the reaction is completed, the crystal of alkaline metal bicarbonate which is formed by the reaction must be removed by filtration or by adding water to dissolve without making the reaction solution acidic. If the intended β-hydroxyketone is not branched at the γ position, or if the remaining material aldehyde is small in amount, the formed bicarbonate crystal may be decomposed with mineral acid such as concentrated hydrochloric acid.

Aldehydes, represented by general formula (A) which can be used in this invention include alkylaldehydes with two α hydrogen atoms and of straight chain or with side chains such as ethanal, propanal, n-butanal, n-caprylaldehyde, 3- methylbutanal, 3-methylhexanal and 3,4-dimethylpentanal; aldehyde substituted by alkylthio group and with two α hydrogen atom such as methylthioethanal, ethylthioethanal and 3-ethylthiobutanal; alkylaldehydes with methyl group at the α position such as isobutanal; alicyclic aldehydes which may have substituents at positions other than the α, β and β' positions such as cyclohexane carbaldehyde, 3-methylcyclohexane carbaldehyde and 4-methylcyclohexane carbaldehyde; heterocyclic aldehydes which may have substituents at positions other than the α, β and β' positions such as 3-tetrahydropyran carbaldehyde, 4-tetrahydropyran carbaldehyde and 3-tetrahydrothiopyran carbaldehyde; and benzaldehydes which may have electron withdrawing groups at the para position such as benzaldehyde, p-nitrobenzaldehyde and p-cyanobenzaldehyde.

The other material, the aqueous solutions of alkali metal salt of acetoacetic acid represented by general formula (B), are aqueous solutions of sodium acetoacetate, potassium acetoacetate or lithium acetoacetate. An aqueous solution of alkali metal salt of acetoacetic acid is easily obtained by hydrolyzing diketene or acetoacetates with aqueous solution of caustic alkali such as sodium hydroxide or potassium hydroxide.

A way of implementing the preparation method of this invention is described in detail in the following.

An aqueous solution containing alkali metal salt of acetoacetic acid of the aforementioned mole ratio to a mole of aldehyde is adjusted the pH to 9 to 10 with mineral acid such as hydrochloric acid or an aqueous solution of caustic alkali, and then aldehyde is added. At this time less than 300ml, to one mole of aldehyde, of water-soluble organic solvent such as methanol may be added. The reaction is carried out with stirring at a reaction temperature of 20 to 70 °C, preferably 40 to 60 °C, for 1 to 9 hours, while keeping the pH to 9 to 10 with aqueous solution of caustic alkali. Organic acids and amines may be used as a base and acid to adjust the pH. Industrially mineral acids or aqueous solutions of caustic alkalis are better. After the reaction is completed, a water-insoluble organic solvent such as chloroform or toluene is added to the obtained reaction mixture, the deposited alkaline metal bicarbonate is filtrated, and the filtrate is separated into aqueous and organic layers according to an ordinary method. The deposited bicarbonate, if small in amount, may be dissolved by adding water. The obtained organic layer is concentrated and distilled under vacuum to give the intended β-hydroxyketone.

This invention is further described in detail by reference to the following examples. The range of this invention is not limited at all by the following examples.

Example 1

Into a reaction vessel of 1 ℓ in inside volume were placed 232.2g (2.0 moles) of methyl acetoacetate and 200.4g of water, to which 293.8g (2.1 moles as NaOH) of 28. 6% NaOH aqueous solution was dropped over an hour with stirring while cooling with water and keeping the inside temperature to below 35°C. The resulting solution was stirred at 35 to 36 °C for 5 hours for hydrolysis. Part of the content in the reaction vessel was

collected to titrate for pH with 1N HCl standard aqueous solution. From the results, the obtained aqueous solution of sodium acetoacetate was 32.9% in concentration and the yield was 97.1% to methyl acetoacetate.

678.9g (1.8 moles as sodium acetoacetate) of the aqueous solution of sodium acetoacetate was placed in a reaction vessel or 2ℓ in inside volume and the pH was adjusted to 10.0 with concentrated hydrochloric acid. Into the vessel were added 198.3g (1.5 moles) of 3-ethylthiobutanal and 300ml of methanol to react with stirring at 50°C for 4 hours. The pH was maintained to 9 to 10 with 28.6% NaOH aqueous solution during the reaction.

After the reaction was completed, the solution was cooled down to room temperature, 750ml of chloroform was added, and the solution was filtrated by aspiration to remove deposited $NaHCO_2$. The filtrate was separated into organic and aqueous layers. The solvent in the obtained organic layer was distilled under reduced pressure. The remaining oily product was distilled under reduced pressure to give 267.2g of colorless oily product with boiling point of 84 to 86°C at 0.2mmHg and $n_D^{22}$ = 1,4850. (Crude yield: 93.6%) The obtained product was analyzed by gas chromatography to find that the intended product, 6-ethylthio-4- hydroxyheptane-2-one was 95.0% in purity. (Yield: 88.9%)

Examples 2 to 8

Example 1 was repeated except using different aldehyde under the conditions shown in Table 1.

The results are shown in Table 1 including that of Example 1.

Example 9

758.9g (2.0 moles as sodium acetoacetate) of 32.7% aqueous solution of sodium acetoacetate, which was synthesized under the same conditions as those used in Example 1. was placed in a reaction vessel of 2ℓ in inside volume and the pH was adjusted to 9.8 with concentrated hydrochloric acid. Into the vessel were added 130.2g (1.0 mole) of 3-tetrahydrothiopyran carbaldehyde and 200ml of methanol to react at 50°C with stirring for 7 hours. The pH was maintained to 9 to 10 with 28.6% NaOH aqueous solution during the reaction.

After the reaction was completed, the solution was cooled down to room temperature, 500ml of chloroform was added, and the solution was filtrated by aspiration to remove deposited $NaNCO_3$. The filtrate was separated into an organic and aqueous layers. The solvent in the obtained organic layer was distilled under reduced pressure. The residue was distilled under reduced pressure to give 177.2g of colorless oily product with boiling point of 98 to 104 °C at 0.2mmHg and $n_D^{23}$ = 1.5223. (Crude yield: 94.1%) The obtained product was analyzed by gas chromatography to find that the intended product, 4-hydroxy-4-(3-tetrahydrothiopyranyl)-butane-2-one was 95.4% in purity. (Yield: 89.8%)

Example 10

Into a reaction vessel of 1ℓ in inside volume were placed 232.2g (2.0 moles) of methyl acetoacetate and 300.3g of water, to which 392.7g (2.1 moles as KOH) of 30% KOH aqueous solution was dropped over 1.5 hours with stirring while cooling with water and keeping the inside temperature to below 35°C. The resulting solution was stirred at 33 to 35 °C for 4 hours for hydrolysis. Part of the content in the reaction vessel was collected to titrate for pH with 1N HCl standard aqueous solution. From the results, the obtained aqueous solution of potassium acetoacetate was 30.8% in concentration and the yield was 96.8% to methyl acetoacetate.

728.2g (1.6 moles as potassium acetoacetate) of the aqueous solution of potassium acetoacetate was placed in a reaction vessel of 2ℓ in inside volume and the pH was adjusted to 9.8 with concentrated hydrochloric acid. Into the vessel were added 130.2g (1.0 moles) of 3-tetrahydrothiopyran carbaldehyde and 200ml of methanol to react with stirring at 40 °C for 9 hours. The pH was maintained to 9 to 10 with 30% KOH aqueous solution during the reaction.

After the reaction was completed, the solution was cooled down to room temperature. 200ml of water was added to dissolve the deposited $KHCO_3$. The liberated oily product was extracted with chloroform, the solvent was distilled under reduced pressure. and the residue was distilled under reduced pressure to give 181.7g of colorless oily product with boiling point of 97 to 104 °C at 0.01mmHg and $n_D^{24}$ = 1.5216. (Crude yield: 96.5%) The obtained product was analyzed by gas chromatography to find that the intended product, 4-hydroxy-4-(3-tetrahydrothiopyranyl)-butane-2-one was 94.8% in purity. (Yield: 91.5%)

Example 11

After the reaction was carried out in the same manner as that used in Example 1, the solution was cooled down to room temperature. Concentrated hydrochloric acid was slowly dropped to make the pH 4.0 to decompose the formed $NaHCO_3$. Methanol was distilled until reaching to distillation temperature of 100 °C. The remaining solution was cooled. The liberated oily product was extracted with 750ml of chloroform, the solvent was distilled under reduced pressure, and the residue was distilled under reduced pressure to give 272.9g of colorless oily product with boiling point of 87 to 93 °C at 0.3mmHg and $n_D^{24}$ = 1.4838. (Crude yield: 95.6%) The obtained product was analyzed by gas chromatography to find that the intended product, 6-ethylthio-4-hydroxyheptane-2-one was 94.4% in purity. (Yield: 90.2%)

Comparative Example 1

Example 11 was repeated except using isobutylaldehyde as the aldehyde. The intended product, 4-hydroxy-5-methyl-2-hexanone was 78.3% in yield.

Comparative Example 2

91.3g (0.24 moles as sodium acetoacetate) of 32.6% aqueous solution of sodium acetoacetate, which was synthesized under the same conditions as tliose used in Example 1, was placed in a 300ml reaction vessel and the pH was adjusted to 9.8 with 10% HCl aqueous solution. Into the vessel were added 40ml of methanol and 26.0g (0.20 mole) of 3-tetrahydrothiopyran carbaldehyde to react at 50 °C with stirring for 6 hours. The pH was maintained to 9 to 10 with 10% NaOH aqueous solution during the reaction.

After the reaction was completed, the solution was cooled down to room temperature. Concentrated hydrochloric acid was slowly dropped to make the pH 4.0 to decompose the formed $NaHCO_3$. Methanol was distilled until reaching to distillation temperature of 100°C. The remaining solution was cooled. The liberated oily product was extracted with 100ml of chloroform. The obtained extract was analyzed by gas chromatography to find that it contained 26.2g of the intended product, 4-hydroxy-4-(3-tetrahydrothiopyranyl)-butane-2-one. This is equivalent to 70.4% of standard yield of 3-tetrahydrothiopyran carbaldehyde.

Comparative Examples 3 to 6

Example 1 was repeated except using 3-tetrahydrothiopyran carbaldehyde as the aldehyde under the conditions shown in Table 2.

The results are shown in Table 2 including those for Comparative Examples 1 and 2.

Table 1

Examples

| Compound No | R - Material aldehyde RCHO | product $\underset{OH}{RCH}CH_2\underset{O}{\overset{\|}{C}}CH_3$ | mole ratio of aqueous solution of sodium acetoacetate to aldehyde | Water soluble organic solvent ml/mole of aldehyde | pH during reaction | Reaction temperature Reaction time | After treatment | Yield (%) to aldehyde | Physical properties |
|---|---|---|---|---|---|---|---|---|---|
| 1 | 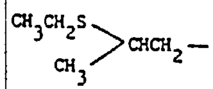 | | 1.2 | methanol 200 | pH9 - 10 | 50°C 4Hr | | 88.9 | b.p. 84 - 86°C(0.2mmHg) $n_D^{22°C}$ 1.4850 |
| 2 | 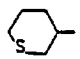 | | 1.6 | " | " | 50°C 6Hr | Same method as that used in Example 1. | 89.2 | b.p. 95 - 98°C(0.008mmHg) $n_D^{21°C}$ 1.5231 |
| 3 | $CH_3(CH_2)_5CH_2$— | | " | " | " | 50°C 6Hr | " | 94.1 | b.p.102 - 1050°C(0.7mmHg) $n_D^{23°C}$ 1.4418 |
| 4 | 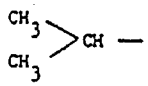 | | " | " | " | 50°C 6Hr | " | 89.7 | b.p.80 - 81°C(10mmHg) $n_D^{10°C}$ 1.4368 |
| 5 | 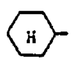 | | " | 1.4-dioxane 200 | " | 50°C 8Hr | " | 90.0 | b.p.95 - 1000°C(1mmHg) $n_D^{21°C}$ 1.4761 |
| 6 | 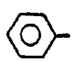 | | 2.0 | ethanol 200 | " | 50°C 6Hr | Same method as that used in Example 1, but toluene was used as extraction solvent | 83.8 | b.p.97 - 99°C(0.5mmHg) $n_D^{17°C}$ 15311 |
| 7 | 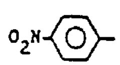 | | 1.4 | — | " | 40°C 3Hr | " | 97.7 | m.p. 59 - 62°C |
| 8 | 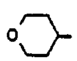 | | 1.5 | methanol 200 | 9 - 10 | 50°C 4Hr | Same method as that used in Example 1 | 91.3 | b.p.118 - 120°C(2mmHg) $n_D^{19°C}$ 1.4837 |

Table 2 Comparative Examples

| Compound No | R - Material aldehyde RCHO | R - product $RCHCH_2CCH_3$<br>OH O | mole ratio of aqueous solution of sodium acetoacetate to aldehyde | Water soluble organic solvent ml/mole of aldehyde | pH during reaction | Reaction temperature<br>Reaction time | After treatment | Yield (%) to aldehyde | * Remark |
|---|---|---|---|---|---|---|---|---|---|
| 1 | 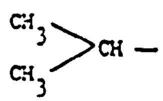 | | 1.2 | methanol<br>200 | pH9 - 10 | 50°C<br>4Hr | Same method as that used in Example 10 | 78.3 | Lower mole ratio of sodium acetoacetate to aldehyde |
| 2 | 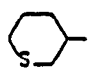 | | " | " | " | 50°C<br>6Hr | " | 70.4 | " |
| 3 | " | | " | " | " | " | Same method as that used in Example 1 | 76.6 | " |
| 4 | " | | " | " | pH8.5 - 9 | " | | 71.9 | Lower mole ratio of sodium acetoacetate to aldehyde and unsuitable pH range |
| 5 | " | | " | " | pH 10.5 | " | | 70.4 | α,β-unsaturated ketone, a dehydrated product of the intended β-hydroxyketone, was by produced 6.8% |
| 6 | " | | " | " | pH12-12.5 | " | | 3.9 | unsuitable pH range |

* The intended product in the organic layer was analyzed by gas chromatography

Industrial Applicability:

According to the method of this invention, intended β-hydroxyketones (Compound 1) can be prepared under mild conditions, safely and easily, and with good yield. This invention is thus extremely significant in industry, particularly in manufacturing of pharmaceuticals and agricultural chemicals.

## Claims

1. Method for the preparation of β-hydroxyketones represented by general formula

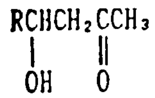

(where R is an alkyl group which may have a methyl group at the α position, an alkyl group substituted by alkylthio group which may have a methyl group at the α position, an alicyclic group which may have substituents at positions other than α, β and β' positions, a heterocyclic group which may have substituents at positions other than the α, β and β' positions, or a phenyl group which may have an electron withdrawing group at the para position), by reacting aldehydes represented by general formula

RCHO (A)

(where R is as defined above) with aqueous solution of alkali metal salt of acetoacetic acid represented by general formula

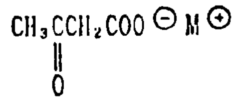 (B)

(where $M^{\oplus}$ is an alkali metal ion), which method comprises reacting (A) and (B) in a mole ratio of (A) to (B) of 1.0 to 1.5 if (A) has two α hydrogen atoms, 1.5 to 2.0 if (A) has one α hydrogen atom, 2.0 to 2.5 if (A) has no α hydrogen atom and 1.1 to 1.5 if R is a phenyl group with electron withdrawing group at the para position at a pH of 9 to 10, without using an amine catalyst.

## Patentansprüche

1. Verfahren zur Herstellung von β-Hydroxyketonen, angegeben durch die allgemeine Formel

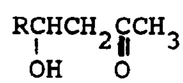

(in der R eine Alkylgruppe ist, die eine Methylgruppe in α-Stellung enthalten kann, eine Alkylgruppe, substituiert durch eine Alkylthiogruppe, die eine Methylgruppe in α-Stellung haben kann, eine alicyclische Gruppe, die Substituenten in anderen als α-, β- und β'-Stellungen enthalten kann, eine heterocyclische Gruppe, die Substituenten in anderen Stellungen als α, β und β' enthalten kann, oder eine Phenylgruppe, die eine Elektronen-abziehende Gruppe in p-Stellung aufweist) durch Umsetzung von Aldehyden, angegeben durch die allgemeine Formel

RCHO (A)

(in der R wie oben definiert ist) mit einer wäßrigen Lösung eines Alkalimetallsalzes von Acetessigsäure, angegeben durch die allgemeine Formel

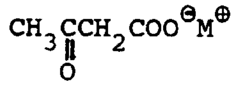 (B)

(in der $M^{\oplus}$ ein Alkalimetallion ist), wobei das Verfahren umfaßt die Umsetzung von (A) und (B) in einem Molverhältnis von (A) zu (B) von 1,0 bis 1,5, wenn (A) zwei α-Wasserstoffatome enthält, 1,5 bis 2,0, wenn (A) ein α-Wasserstoffatom enthält, 2,0 bis 2,5, wenn (A) kein α-Wasserstoffatom enthält, und 1,1 bis 1,5, wenn R eine Phenylgruppe mit einer Elektronen-abziehenden Gruppe in p-Stellung ist, bei einem pH-Wert von 9 bis 10, ohne Anwendung eines Aminkatalysators.

## Revendications

1. Procédé pour la préparation de β-hydroxycétones représentées par la formule générale

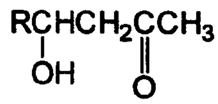

(où R est un groupe alkyle qui peut avoir un groupe méthyle en position α, un groupe alkyle substitué par un groupe alkylthio qui peut avoir un groupe méthyle en position α, un groupe alicyclique qui peut avoir des substituants aux positions autres que les positions α, β et β', un groupe hétérocyclique qui peut avoir des substitutions aux positions autres que les positions α, β et β', ou un groupe phényle qui peut avoir un groupe électro-attracteur en position para), par réaction d'aldéhydes représentées par la formule générale

RCHO (A)

(où R est tel que défini ci-dessus) avec une solution aqueuse d'un sel de métal alcali d'acide acétoacétique représenté par la formule générale

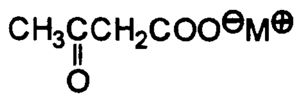

(B)

(où $M^{\ominus}$ est un ion de métal alcali), lequel procédé comprend la réaction de (A) et (B) dans un rapport molaire de (A) à (B) de 1,0 à 1,5 si (A) a deux atomes d'hydrogène α, de 1,5 à 2,0 si (A) a un atome d'hydrogène α, de 2,0 à 2,5 si (A) n'a pas d'atome d'hydrogène α de 1,1 à 1,5 si R est un groupe phényle avec un groupe électro-attracteur en position para à un pH de 9 à 10, sans utiliser un catalyseur amine.